Europäisches Patentamt

**European Patent Office**

**Office européen des brevets**

(11) Veröffentlichungsnummer: **0 500 983 B1**

(12) **EUROPÄISCHE PATENTSCHRIFT**

(45) Veröffentlichungstag der Patentschrift: **26.07.95**

(51) Int. Cl.6: **A61N 1/40**

(21) Anmeldenummer: **91102900.7**

(22) Anmeldetag: **28.02.91**

(54) **Bestrahlungsvorrichtung zur Behandlung von lebendem Gewebe mit elektromagnetischen Wellen.**

(43) Veröffentlichungstag der Anmeldung:
**02.09.92 Patentblatt 92/36**

(45) Bekanntmachung des Hinweises auf die
Patenterteilung:
**26.07.95 Patentblatt 95/30**

(84) Benannte Vertragsstaaten:
**AT BE CH DE DK ES FR GB GR IT LI LU NL SE**

(56) Entgegenhaltungen:
**EP-A- 0 132 051**
**EP-A- 0 136 530**
**US-A- 4 911 686**

(73) Patentinhaber: **MEDI-LINE GMBH**
**Manfred-von-Richthofen-Strasse 15**
**D-12101 Berlin (DE)**

(72) Erfinder: **Broers, Dieter**
**Sredzkistrasse 47**
**W-1058 Berlin (DE)**

(74) Vertreter: **Neubauer, Hans-Jürgen, Dipl.-Phys.**
**et al**
**Fauststrasse 30**
**D-85051 Ingolstadt (DE)**

## Beschreibung

Die Erfindung betrifft ein Gerät zur Behandlung von lebendem Gewebe und Organen mit elektromagnetischen Wellen zur therapeutischen Beeinflussung bei Erkrankungen bakterieller, viröser und nervlicher Art sowie bei Tumorbildung gemäß dem Oberbegriff des Anspruchs 1.

In Versuchen wurde festgestellt, daß es elektromagnetische Wellen in Zellen gibt. Diese Strahlung war, von wenigen Ausnahmen abgesehen, z.B. bei einigen Algen, Bakterien, Protozoen, in allen untersuchten, pflanzlichen und tierischen Geweben nachzuweisen, auch dem menschlichen Gewebe. Die Intensität liegt stets in der ebenfalls im Versuch ermittelten Größenordnung von einigen 100 bis 1.000 Photonen pro Sekunde und pro cm² Zellkulturfläche. Die Energien der Photonen erstreckten sich auf den Wellenbereich von 800 bis etwa 350 Nanometer mit einem Maximum bei ca. 550 Nanometer.

Es wird davon ausgegangen, daß diese Strahlung auch zur Kommunikation zwischen den Zellen dient und beispielsweise auch das Wachstum reguliert. Ein Hinweis darauf ist die starke Änderung in der Intensität und im Spektrum der Strahlung von Krebsgewebe. Krebszellen sind somit als resonanzgestörte Zellen anzusehen, deren chronische Resonanzstörung zu dauerhaftem Wachstum führt. Das verstärkte Wachstum führt zu einer noch stärkeren Resonanzstörung, so daß eine Selbstgeneration nicht mehr möglich ist. Es ist auch ohne weiteres denkbar, daß eine gesunde Zelle durch eine Strahlung von Mikroben aus dem Schwingungsgleichgewicht gebracht wird und damit krank wird.

Beobachtungen haben gezeigt, daß der Krebs in der Mehrzahl der Fälle bei Personen mittleren Alters - etwa von 50 Jahren an - und bei Greisen auftritt. Daraus kann geschlossen werden, daß er sich in "abgenutztem" Gewebe ausbildet. Es ist daher anzunehmen, daß die Anfälligkeit für Krebs in einer Veränderung des Blutes oder der Zellen besteht, die sich mit jenen Altersstufen einstellt bzw. durch die Veränderungen der zellulären Schwingungen, die durch die Veränderung der Kapazität der Zellen bewirkt wird.

Ausgehend von solchen Beobachtungen sind Bestrahlungsvorrichtungen, die mit elektromagnetischen Wellen arbeiten, bereits bekannt.

Für eine Zerstörung von Tumorgewebe werden seit langem ionisierende, elektromagnetische Strahlen angewendet. Eine solche Behandlung hat den Nachteil, daß nicht nur das erkrankte Gewebe bestrahlt wird, sondern auch gesundes Gewebe, durch das die Strahlung hindurchgeht, geschädigt wird, da die hier verwendete Strahlung relativ hart und energiereich ist.

Bei einer anderen, bekannten Behandlungsmethode von Tumoren (DE-B 26 34 628, DE-B 23 04 500, DE-B 23 06 922) wird das gegenüber dem gesunden Gewebe schon wärmere Tumorgewebe so überhitzt, daß dieses zerstört wird. Auch bei einer solchen Behandlung wird gesundes Gewebe geschädigt, insbesondere treten schmerzhafte Verbrennungen der Hautpartien auf, durch die die elektromagnetischen Wellen zugeführt werden.

Es ist bekannt (DE-B 27 48 780), durch eine Änderung der elektrischen oder elektrochemischen Umgebung einer lebenden Zelle oder eines Gewebes einen therapeutischen Effekt zu erzielen.

Eine weitere Bestrahlungsvorrichtung, die mit elektromagnetischen Strahlen arbeitet, ist aus der EP-A1-0 011 019 bekannt, die zur Beeinflussung der Zellenaktivität dient. Bei dieser Einrichtung (Fig. 1) wird ein Hochfrequenzgenerator eingesetzt, der mit einer Frequenz von 27,12 MHz schwingt. Dem Hochfrequenzoszillator nachgeschaltet ist ein Verstärker, an den ein Taktgenerator (Modulationsgenerator) angeschlossen ist. Der Taktgenerator erzeugt im wesentlichen Rechteckimpulse, die dazu dienen, den Ausgang des Verstärkers periodisch zu unterbrechen. Dadurch werden dem nachgeschalteten Leistungsverstärker bzw. einer angeschlossenen Antenne zeitlich unterbrochene Wellenzugstücke zugeführt. Als charakteristische Werte für die Taktfrequenz werden dort 50 bis 1.000 MHz genannt, wobei die Impulsdauer bzw. Dauer eines Wellenzugs ca. 10 bis 100 Mikrosekunden lang sein soll. Da feststeht, daß es elektromagnetische Wellen in Zellen gibt und andererseits durch Bestrahlung mit elektromagnetischen Wellen die Zellen auch beeinflußbar sind, wird der bekannten Einrichtung eine Zellbeeinflussung nicht abgesprochen. Aufgrund intensiver Versuche und theoretischer Überlegungen hat sich allerdings gezeigt, daß eine Bestrahlungseinrichtung für eine effektive Therapie für andere Frequenzbereiche ausgelegt sein muß.

In einer bekannten, gattungsgemäßen Bestrahlungsvorrichtung (EP 0 136 530 B1) zur Behandlung von lebendem Gewebe mit elektromagnetischen Wellen ist eine Hochfrequenzoszillatorstufe vorgesehen, die in einem einstellbaren Frequenzbereich von 100 bis 200 MHz arbeitet. Diese Frequenz wird mit einer von 1 Hz bis 1.000 Hz einstellbaren Niederfrequenz moduliert und einem Taktgenerator zugeführt, der mit einer Taktfrequenz von 0,5 Hz bis 40 Hz einstellbar arbeitet. Die Wellenzugteile werden einem Endverstärker zugeführt, an den eine Sendeantenne angeschlossen ist. Im Abstrahlbereich der Sendeantenne wird das zu behandelnde Gewebe angebracht oder liegt der Patient. Die Antenne kann auch in das zu bestrahlende Gewebe implantiert sein.

Die vorstehend beschriebene Bestrahlungsvorrichtung wurde bereits in einer Vielzahl von Geräten hergestellt und angewendet. Dabei hat sich gezeigt, daß eine Bestrahlung mit der hochfrequenten Grundfrequenz von 100 MHz bis 200 MHz als Trägerfrequenz, die mit Niederfrequenz moduliert ist, ein hervorragender, therapeutischer Effekt erzielbar ist. Auch die Zerhackung in Wellenzugteile ist therapeutisch wertvoll. Mit einer solchen Bestrahlungsvorrichtung werden somit elektromagnetische Schwingungen mit für therapeutische Zwecke geeigneter Frequenz und Amplitude verfügbar, die die Gesundheit fördern und wiederbringen können.

Es kann damit angenommen werden, daß mit einer solchen Bestrahlungsvorrichtung biologische Reaktionen hervorgerufen werden, wobei die erzeugten, modulierten, elektromagnetischen Wellen mit den Grundbestandteilen einer gesunden Zelle in Resonanz stehen. Dadurch wird ein magnetisches Wechselfeld im Körper des Patienten erzeugt, dessen Wirbelströme günstige Ladungsverschiebungen in den Zellmembranen zur Folge haben und im vegetativen Nervensystem günstige Reizungen hervorrufen, die vorhandene Blockierungen abbauen. Alle diese Einflüsse laufen insgesamt auf eine Stärkung des Immunsystems hinaus.

Dazu sind vorteilhaft nur elektromagnetische Wellen mit einer sehr geringen Leistung im Milliwatt- bis ca. 10 Watt-Bereich erforderlich, die die angeführten biologischen Wirkungen bereits hervorrufen, so daß eine Strahlungsschädigung des Patienten im gesunden Gewebe praktisch ausgeschlossen ist.

Der energiereiche Hochfrequenzanteil der modulierten Schwingung dient im wesentlichen als Trägerfrequenz, die ein Eindringen in das lebende Gewebe ermöglicht; eine patientenspezifische Einstellung innerhalb des Frequenzbereichs von 100 MHz bis 200 MHz ist für einen guten therapeutischen Effekt nicht in hohem Maße ausschlaggebend. Anders hat sich dies für den niederfrequenten Anteil erwiesen, der in einem Bereich von 1 Hz bis 1.000 Hz einstellbar ist. Für einen guten, therapeutischen Effekt ist es hier wesentlich, die Bestrahlung mit einer patientenspezifischen Frequenz durchzuführen. Es hat sich gezeigt, daß ein therapeutischer Effekt bei unterschiedlichen Patienten mit unterschiedlichen Bestrahlungsfrequenzen auftritt.

Dieser Erkenntnis wird bereits in einem Teilbereich der oben genannten, gattungsgemäßen Bestrahlungsvorrichtung (EP 0 136 530 B1) Rechnung getragen: Es ist eine zweite Niederfrequenzoszillatorstufe vorgesehen, die eine im Bereich von 1 Hz bis 1.000 Hz einstellbare Frequenz erzeugt und die als magnetische Strahlung gleichzeitig mit der elektromagnetischen Strahlung abgestrahlt wird. Diese zweite Niederfrequenzoszillatorstufe ist als Wellenschaukel ausgebildet, wobei die Frequenz kontinuierlich im Frequenzbereich ständig verändert und hin- und hergefahren wird. Damit soll erreicht werden, daß beim Bestrahlungsvorgang keine feste Niederfrequenz vorgegeben wird, auf die der Patient möglicherweise nicht oder nur mit geringem Effekt anspricht, sondern daß in jedem Fall zumindest kurzzeitig die patientenspezifische Frequenz mit hoher Ansprechempfindlichkeit und hohem, therapeutischen Effekt überfahren wird.

Ersichtlich wird mit dieser Maßnahme nur jeweils für den kurzen Zeitraum des Überstreichens die hochwirksame Bestrahlungsfrequenz abgegeben. Für die übrige Zeit ist die Bestrahlung weniger wirksam, so daß eine kontinuierliche, ununterbrochene Beeinflussung des Patienten mit der patientenspezifischen, hoch wirksamen Bestrahlungsfrequenz nicht gegeben ist. Zudem wird lediglich davon ausgegangen, daß eine patientenspezifische, hoch wirksame Bestrahlungsfrequenz im Wellenschaukelbereich vorhanden ist. Wo diese für den konkreten Patienten für den jeweiligen Bestrahlungsvorgang liegt, kann mit der bekannten Bestrahlungsvorrichtung weder erfaßt werden noch ist ein Hinweis auf eine automatische Einstellung des Geräts gegeben.

Ausgehend vom letztgenannten Stand der Technik wird daher die Aufgabe der Erfindung darin gesehen, eine gattungsgemäße Bestrahlungsvorrichtung so weiterzubilden, daß damit ein größerer therapeutischer Effekt erzielbar ist.

Diese Aufgabe wird mit den Merkmalen des Anspruchs 1 gelöst.

Gemäß Anspruch 1 enthält die Bestrahlungsvorrichtung eine Hochfrequenzoszillatorstufe mit einem einstellbaren Frequenzbereich von 100 bis 200 MHz. Weiter enthält die Bestrahlungsvorrichtung eine Niederfrequenzoszillatorstufe mit einem einstellbaren Frequenzbereich von 1 Hz bis 100 KHz, bevorzugt bis 10 KHz. Die Ausgänge dieser Hochfrequenzoszillatorstufen sind in einer Modulationseinheit zusammengeführt, so daß ein modulierter Wellenzug nach der Modulationseinheit vorliegt, der einem Endverstärker zugeführt wird. Am Endverstärker ist eine Sendeantenne anschließbar, in derem Abstrahlbereich das zu behandelnde Gewebe bzw. ein Patient postierbar ist.

Erfindungsgemäß ist die Frequenzeinstellung der Niederfrequenzoszillatorstufe mit Hilfe einer angeschlossenen Steuerheit steuerbar ausgeführt.

Über wenigstens einen, an sich bekannten Sensor sind patienteneigene Schwingungen aufnehmbar, wobei dem Sensor ein Signalanalysator zur Bewertung dieser Schwingungen nachgeschaltet ist. Der Signalanalysator ist insbesondere so ausgelegt, daß er Änderungen eines Sensorsignals bzw. Änderungen in den patienteneigenen Schwingungen hinsichtlich Frequenz, Oberwellenanteil und/oder Amplitude erfassen kann.

Die Steuereinheit zur Steuerung der Frequenzeinstellung der Niederfrequenzoszillatorstufe ist so ausgelegt, daß nach einem Start ein (teilweiser) Frequenzdurchlauf durch den Niederfrequenzbereich von 1 Hz bis 100 KHz ausgeführt wird. Dieser Frequenzdurchlauf kann bevorzugt von unten her (1 Hz) oder auch von oben her (100 KHz) beginnend ausgeführt werden. Zweckmäßigerweise wird dieser Durchlauf durch einen Zeitgeber gesteuert, der die Frequenzen nacheinander oder um einen bestimmten Sprung mit anschließenden Wartezeiten für eine Beobachtung der Patientenreaktion (verbunden mit einer Änderung des Sensorsignals) weiterschaltet.

Der Signalanalysator gibt bei einer Änderung des Sensorsignals, was einem Ansprechen des Patienten auf eine bestimmte, gerade abgestrahlte Frequenz im Frequenzdurchlauf entspricht, ein Haltesignal an die angeschlossene Steuereinheit ab. Diese stoppt nach Empfang des Haltesignals den Frequenzdurchlauf, so daß die gerade anliegende Frequenz für den weiteren Bestrahlungsvorgang beibehalten wird.

Damit wird in vorteilhafter Weise automatisch von der Bestrahlungsvorrichtung in der Art einer Rückkopplung über den Patienten eine Frequenzeinstellung im Niederfrequenzbereich gefunden, auf die der Patient anspricht und die damit einen hohen therapeutischen Effekt erwarten läßt.

Als Sensoren für geeignete, patienteneigene Schwingungen können nach Anspruch 2 EEG-Sensoren (EEG = Elektroenzephalogramm), EKG-Sensoren (EKG = Elektrokardiogramm) oder Hautwiderstandssensoren zur Messung des elektrischen Hautwiderstandes verwendet werden. Mit jedem dieser Sensoren können Schwingungen ermittelt werden, über die erfindungsgemäß die Niederfrequenzeinstellung der Bestrahlungsvorrichtung erfolgen kann. Grundsätzlich sind auch andere Sensoren verwendbar, z.B. pH-Sensoren, zur Erfassung einer Reizantwort.

In der Ausführung nach Anspruch 1 wird die Einstellung der Niederfrequenz durch Rückkopplung über den Patienten einmal zu Beginn des Bestrahlungsvorgangs durchgeführt. Da insbesondere bei längeren Bestrahlungsdauern eine Veränderung der optimalen Ansprechfrequenz des Patienten möglich ist, wird mit Anspruch 3 vorgeschlagen, den Frequenzdurchlauf mit anschließendem Festhalten einer optimalen Ansprechfrequenz in gewissen Abständen während der gesamten Bestrahlungsdauer durchzuführen. Damit wird automatisch jeweils auf eine optimale Ansprechfrequenz bei einer Veränderung nachreguliert.

Es hat sich gezeigt, daß der Therapieerfolg gesteigert werden kann, wenn gemäß Anspruch 4 der modulierte Wellenzug über einen Taktgenerator in Wellenzugteile zerhackt wird. Eine einstellbare Taktfrequenz von 0,5 Hz bis 40 Hz hat sich als gut geeignet erwiesen, wobei das Verhältnis zwischen Wellenzugdauer und Pausenzeit nach Anspruch 5 1:1 oder kleiner sein soll.

Mit den Merkmalen des Anspruchs 6 wird einerseits eine Anpassung der Sendeantenne an die Sendefrequenz erreicht und andererseits die Möglichkeit geschaffen, unterschiedlich lange Antennen (Halsband, Hüftgürtel, usw.) einzusetzen.

In Anspruch 7 ist der bevorzugte Frequenzbereich angegeben, bei dem eine noch gesteigerte Wirkung erkannt wurde, so daß ggfs. die Bestrahlungseinrichtung nur auf diesen Frequenzbereich ausgelegt sein könnte.

Mit den Merkmalen des Anspruchs 8 wird eine vorteilhafte Ausgestaltung und Weiterbildung des Erfindungsgegenstandes angegeben, wodurch eine weitere Steigerung der therapeutischen Wirkung zu erreichen ist. Dazu wird eine weitere Niederfrequenzoszillatorstufe vorgesehen, die von 1 Hz bis 100 KHz, bevorzugt bis 10 KHz, einstellbar ist und die mit einer Spule als Abstrahlelement verbunden ist. Die Spule und zugleich die Sendeantenne sind bei einer Behandlung auf das zu behandelnde Gewebe oder einem Patienten zu richten. Auch diese zweite Niederfrequenzoszillatorstufe kann entsprechend der vorstehenden, erfindungsgemäßen Merkmale durch die Rückkopplung über den Patienten auf eine als optimal erkannte Ansprechfrequenz automatisch einstellbar gestaltet werden.

Die Sendeleistung der Bestrahlungseinrichtung kann innerhalb der in Anspruch 9 genannten, sehr kleinen Werte liegen, bei denen eine Gewebeschädigung ausgeschlossen ist. Diese geringen Bestrahlungsleistungen (0,5 mW bis 100 mW, bevorzugt 10 mW) sind wesentlich, da sich gezeigt hat, daß die Effektivität bei höheren Leistungen wieder abnimmt.

Mit den Merkmalen des Anspruchs 10 wird eine weitere Verbesserung der Handhabung und Automatisierung der Bestrahlungsvorrichtung erreicht.

Nach Anspruch 11 ist der Signalanalysator so ausgelegt, daß er patienteneigene, unregelmäßige Schwingungen mit hohem Oberwellenanteil als pathologische Schwingungen erfassen und erkennen kann. Solche pathologischen Schwingungen werden ausgefiltert und einer Invers-Schaltung zugeleitet, in der sie invertiert und ggfs. nach einer weiteren Verstärkung dem Patienten sofort wieder in Form von inversen Bestrahlungsschwingungen zugeführt werden. Damit werden die pathologischen Schwingungen am Patienten praktisch ausgelöscht. Es ist davon auszugehen, daß damit die für eine positive Beeinflussung zusätzlich von der Bestrahlungsvorrichtung abgestrahlten Schwingungen und Frequenzen in ihrer therapeutischen Wirkung verbessert werden.

In einer zeitlichen Abfolge kann es somit auch vorteilhaft sein, nach dem Start der Bestrahlungsvorrichtung am Patienten zuerst die Auslöschung pathologischer Schwingungen vorzunehmen und erst dann den Frequenzdurchlauf durchzuführen.

In einer bevorzugten Ausführungsform nach Anspruch 12 erfaßt und erkennt der Signalanalysator patienteneigene, regelmäßige Schwingungen als gesunde Schwingungen. Diese werden der Steuereinheit zugeleitet. Die Steuereinheit regelt dann die eingestellte Niederfrequenz entsprechend der Frequenz und ggfs. der Amplitude der gesunden Schwingung nach. Damit wird eine Verstärkung der gesunden Schwingung über die Bestrahlungsvorrichtung erreicht.

Anhand einer Zeichnung wird die Erfindung näher erläutert.

Es zeigen

Fig. 1 ein Blockschaltbild einer Bestrahlungsvorrichtung,

Fig. 2 ein Flußdiagramm zur Erläuterung der Funktion der Bestrahlungsvorrichtung und

Fig. 3 eine Darstellung einer patienteneigenen Schwingung.

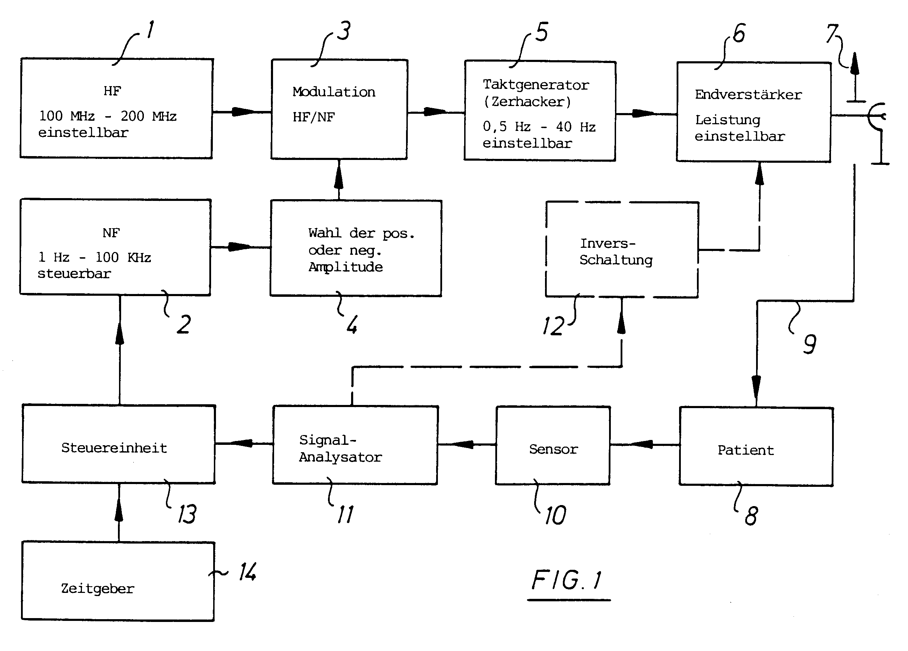

In Fig. 1 ist ein Blockschaltbild einer erfindungsgemäßen Bestrahlungsvorrichtung zur Behandlung von lebendem Gewebe mit elektromagnetischen Wellen dargestellt, wobei ein Hochfrequenzoszillator 1 und ein Niederfrequenzoszillator 2 parallel liegen. Ihre Ausgänge sind in einer Modulationseinheit 3 zusammengeführt, wobei in einer Einheit 4 eine Wahl der positiven oder negativen Amplitude des Niederfrequenzoszillators 2 erfolgen kann. Der Hochfrequenzoszillator 1 hat einen Einstellbereich von 100 MHz bis 200 MHz. Der Niederfrequenzoszillator 2 ist in einem Frequenzbereich von 1 Hz bis 100 KHz steuerbar.

Der modulierte Wellenzug nach der Modulationseinheit 3 wird von einem Taktgenerator 5 mit einem einstellbaren Bereich von 0,5 Hz bis 40 Hz zerhackt und einem Endverstärker 6 zugeführt. Am Endverstärker 6 ist eine Sendeantenne 7 zur Abstrahlung der elektromagnetischen Wellenzugteile angeschlossen.

Mit dem Bezugzeichen 8 ist schematisch ein Patient dargestellt, auf den die abgestrahlten, elektromagnetischen Wellen (dargestellt durch den Pfeil 9) einwirken. Am Patient ist ein Sensor 10 angebracht zur Aufnahme von patienteneigenen Schwingungen nach Frequenz und Amplitude in einem Elektro-Enze-Phalogramm (EEG). Eine typische Grundschwingung liegt für einen gesunden Menschen hier bei z.B. 200 Hz mit einem relativ gleichmäßigen Signalverlauf in Frequenz und Intensität und ohne starke Frequenzänderungen. Pathologische Schwingungen sind gekennzeichnet durch Abweichungen und Änderungen in diesen Merkmalen, z.B. hohe Oberwellenanteile, starke Frequenzänderungen, zum Teil mit Aussetzern, usw.

Dem Sensor 10 nachgeschaltet ist ein Signalanalysator 11, mit dem Änderungen der Sensorsignale erfaßbar und bewertbar sind. Insbesondere ist eine globale Veränderung der Sensorsignale im Sinne eines Ansprechens des Patienten auf eine durchgeführte Bestrahlung erfaßbar. Weiter sind für die weiterführende Ausführung mit einer nachgeordneten Invers-Schaltung pathologische Schwingungen erkennbar und erfaßbar.

Der Signalanalysator ist mit einer Steuereinheit 13 verbunden, die für eine steuerbare Einstellung der Frequenz mit dem Niederfrequenzoszillator 2 verbunden ist. An die Steuereinheit 13 ist weiter ein Zeitgeber 14 zur Vorgabe verschiedener, zeitversetzter Frequenzdurchläufe angeschlossen.

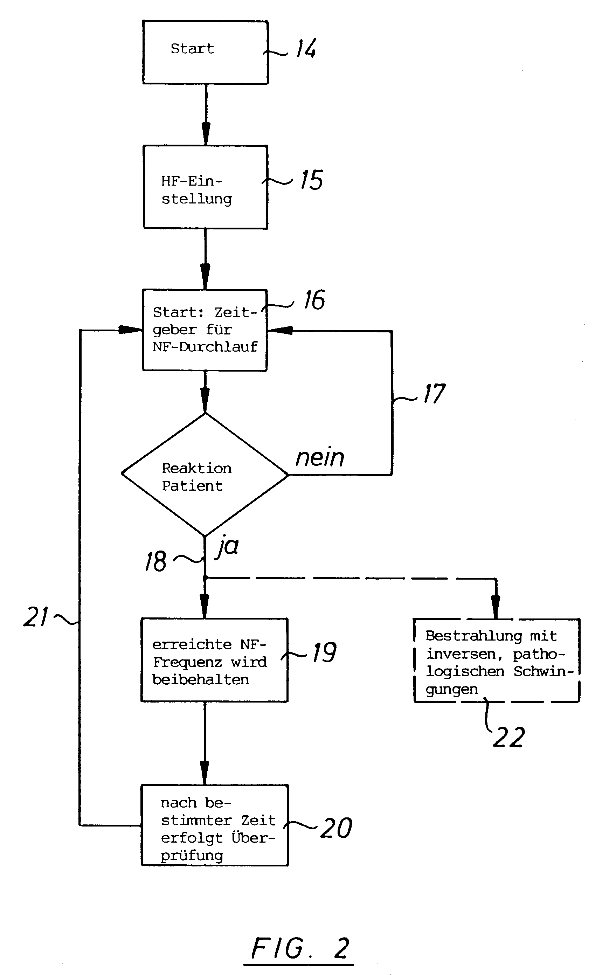

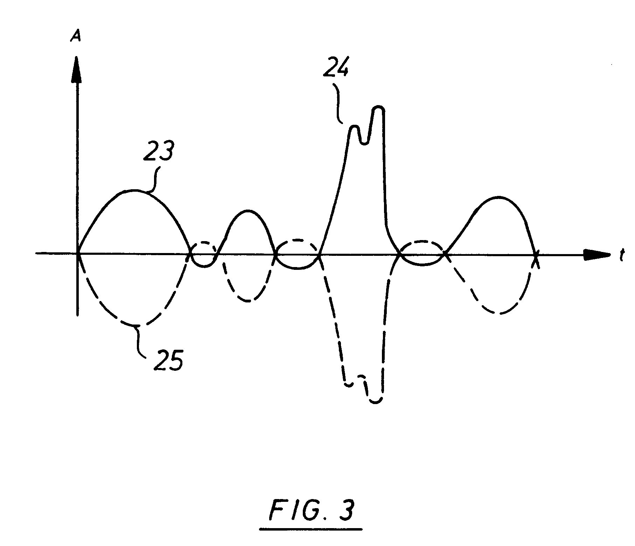

Anhand des Flußdiagramms nach Fig. 2 und der in Fig. 3 dargestellten Schwingungskurve wird die Funktion der im Blockschaltbild 1 dargestellten Bestrahlungsvorrichtung näher erläutert.

Der Start der Bestrahlungsvorrichtung gemäß Operationsblock 14 erfolgt durch Einschalten der Versorgungsspannung oder über einen separaten Start-Schalter. Im Operationsblock 15 ist das Erfordernis der Frequenzeinstellung im Hochfrequenzoszillator dargestellt. Diese Einstellung erfolgt nach Erfahrungswerten und betrifft im wesentlichen die für die Eindringtiefe erforderliche Energie der Trägerfrequenz, die auch im Zusammenhang mit der Abstrahlleistung einzustellen ist.

Anschließend wird der Zeitgeber 14 gestartet, wonach über die Steuereinheit 13 ein (teilweiser) Frequenzdurchlauf im Niederfrequenzbereich von 1 Hz bis 100 KHz durchgeführt wird.

Zweckmäßig wird mit der tiefsten Frequenz von 1 Hz begonnen und die Frequenz ggfs. in bestimmten Zeitabschnitten nach oben gezogen. Entsprechend der im Frequenzdurchlauf gerade anliegenden Frequenz wird der Patient 8 bestrahlt. Über den Sensor 10 und den Signalanalysator 11 wird nun festgestellt, ob eine merkliche Änderung in der abgenommenen, patienteneigenen EEG-Schwingung beispielsweise in der Form einer Amplitudenerhöhung oder Frequenzänderung erfolgt.

Ist keine merkliche Änderung und damit keine Reaktion des Patienten auf die Bestrahlung mit der vorgegebenen Niederfrequenz feststellbar (Zweig 17), wird der Frequenzdurchlauf zu höheren Frequenzen fortgesetzt.

Wird dagegen eine Änderung und damit eine Rekation des Patienten auf die gerade vorliegende Niederfrequenz festgestellt, gibt der Signalanalysator 11 einen Befehl in Form eines Haltesignals an die Steuereinheit 13 ab, wodurch der Frequenzdurchlauf gestoppt und die gerade anliegende Niederfrequenz für den weiteren Bestrahlungsvorgang beibehalten wird (Operationsblock 19).

Mit Operationsblock 20 wird die Funktion einer verbesserten Ausführungsform dargestellt, in der nach einer bestimmten, beispielsweise am Zeitgeber 14 einstellbaren Zeit während der Bestrahlungsdauer eine Überprüfung der als optimal erkannten und beibehaltenen Niederfrequenz erfolgt, indem ein erneuter Frequenzdurchlauf gestartet wird (Zweig 21). Zwischenzeitlich eingetretene Veränderungen hinsichtlich der optimalen Bestrahlungsfrequenz werden dadurch erkannt und nachreguliert.

Mit einer im Operationsblock 22 angeführten, zusätzlichen Bestrahlung des Patienten mit inversen, im Signalanalysator 11 ermittelten und in der Invers-Schaltung 12 invertierten Schwingungen kann eine weitere Verbesserung des therapeutischen Effekts erwartet werden, da der Patient nach einem Auslöschen der pathologischen Schwingungen hinsichtlich dieser Störungen in eine Neutralphase versetzt wird.

In Fig. 3 ist dazu beispielhaft eine patienteneigene Schwingung 23 dargestellt, mit einer Unregelmäßigkeit 24, die auf eine pathologische Schwingung hindeutet. Diese Schwingung wird somit von dem Signalanalysator 11 ausgesondert und der Invers-Schaltung 12 zugeführt, wodurch die (strichliert eingezeichnete) invertierte Schwingung 25 von der Bestrahlungseinheit erzeugt und sofort dem Patienten als elektromagnetische Welle über die Sendeantenne 7 zugeführt wird. Dadurch erfolgt im Sinne der Wellentheorie eine Summierung der Amplituden und damit eine Auslöschung der als pathologisch erkannten Schwingung 23 im Patienten. Durch Auslöschung solcher pathologischer Schwingungen kann erwartet werden, daß die positive Wirkung der anderen abgestrahlten Frequenzen für einen hohen therapeutischen Effekt verbessert wird.

**Patentansprüche**

1. Bestrahlungsvorrichtung zur Behandlung von lebendem Gewebe mit elektromagnetischen Wellen,
mit einer Hochfrequenzoszillatorstufe zur Erzeugung eines Hochfrequenzwellenzuges, wobei die Hochfrequenzoszillatorstufe eine im Bereich von 100 bis 200 MHz einstellbare Frequenz erzeugt,
mit einer Niederfrequenzoszillatorstufe, die eine einstellbare Frequenz erzeugt,
mit einer Modulationseinheit, der der Ausgang der Hochfrequenzoszillatorstufe und der Ausgang der Niederfrequenzoszillatorstufe zugeführt ist, so daß ein modulierter Wellenzug entsteht und
mit einem Endverstärker, an den eine Sendeantenne anschließbar ist, in derem Abstrahlbereich das zu behandelnde Gewebe bzw. ein Patient postierbar ist,
dadurch gekennzeichnet,
daß die Frequenzeinstellung der Niederfrequenzoszillatorstufe (2) in einem Frequenzbereich von 1 Hz bis 100 KHz, bevorzugt bis 10 KHz, durch eine angeschlossene Steuereinheit (13) steuerbar ausgeführt ist,
daß über wenigstens einen an sich bekannten Sensor (10) patienteneigene Schwingungen (23) aufnehmbar sind,
daß dem Sensor (10) ein Signalanalysator (11) zur Bewertung der patienteneigenen Schwingungen (23) nachgeschaltet ist, der insbesondere Änderungen dieser Schwingungen erfaßt,
daß die Steuereinheit (13) nach einem Start der Bestrahlungsvorrichtung einen (teilweisen) Frequenzdurchlauf (Operationsblock 16) von unten oder oben beginnend durch den Niederfrequenzbereich ggfs. über einen Zeitgeber (14) gesteuert ausführt,
daß der Signalanalysator (11) mit der Steuereinheit (13) verbunden ist und bei einer festgestellten Änderung der Schwingungen, was einem Ansprechen des Patienten (8) auf eine bestimmte, gerade abgestrahlte Frequenz im Frequenzdurchlauf entspricht, ein Haltesignal an die Steuereinheit (13) abgibt und
daß die Steuereinheit (13) nach Empfang des Haltesignals den Frequenzdurchlauf stoppt, so daß die gerade anliegende Frequenz für den weiteren Bestrahlungsvorgang beibehalten wird.

2. Bestrahlungsvorrichtung nach Anspruch 1, dadurch gekennzeichnet, daß als Sensoren (10) zur Erfassung von patienteneigenen Schwingungen EEG-Sensoren, EKG-Sensoren oder Hautwiderstands-Sensoren verwendet werden, die die Schwingungen nach Frequenz und Amplitude erfassen.

3. Bestrahlungsvorrichtung nach Anspruch 1 oder 2, dadurch gekennzeichnet, daß während einer Bestrahlungsdauer zur Überprüfung der festgehaltenen Bestrahlungsfrequenz durch den Zeitgeber (14) erneut ein oder ggfs. mehrere Frequenzdurchläufe nacheinander durchgeführt werden (Operationsblock 20), so daß mögliche Veränderungen in der optimalen Ansprechfrequenz des Patienten (8) während des Bestrahlungsvorgangs berücksichtigt und automatisch nachreguliert werden.

4. Bestrahlungsvorrichtung nach einem der Ansprüche 1 bis 3, dadurch gekennzeichnet, daß zwischen die Modulationseinheit (3) und den Endverstärker (6) ein Taktgenerator (5) als Zerhacker geschaltet ist, so daß der modulierte Wellenzug dem Taktgenerator (5) zugeführt wird und der Taktgenerator (5) in einer Taktfrequenz von 0,5 Hz bis 40 Hz einstellbar ist.

5. Bestrahlungsvorrichtung nach Anspruch 4, dadurch gekennzeichnet, daß das Verhältnis zwischen der Dauer des am Taktgenerator (5) durchgelassenen Wellenzuges zu der Pausenzeit 1:1 oder kleiner ist.

6. Bestrahlungsvorrichtung nach einem der Ansprüche 1 bis 5, dadurch gekennzeichnet, daß die Länge der Sendeantenne (7) der eingestellten Hochfrequenz bzw. deren Wellenlänge λ oder ganzen Teilen wie $\frac{\lambda}{2}$ , $\frac{\lambda}{4}$ etc. entspricht.

7. Bestrahlungsvorrichtung nach einem der Ansprüche 1 bis 6, dadurch gekennzeichnet, daß die Hochfrequenzoszillatorstufe (1) eine Frequenz von 140 bis 160 MHz erzeugt.

8. Bestrahlungsvorrichtung nach einem der Ansprüche 1 bis 7, dadurch gekennzeichnet, daß eine zweite Niederfrequenzoszillatorstufe enthalten ist, die eine im Bereich von 1 Hz bis 100 KHz, bevorzugt bis 10 KHz, einstellbare Frequenz erzeugt, daß der zweiten Niederfrequenzoszillatorstufe ein zweiter Endverstärker nachgeschaltet ist, an den eine Spule als Abstrahlelement anschließbar ist, wobei das zu behandelnde Gewebe bzw. ein Patient gleichzeitig in den Abstrahlbereich der Sendeantenne (7) und in den Abstrahlbereich der Spule bringbar ist.

9. Bestrahlungsvorrichtung nach einem der Ansprüche 1 bis 8, dadurch gekennzeichnet, daß die Sendeleistung am Endverstärker (6) von 0,5 mW bis 100 mW, bevorzugt 10 mW, einstellbar ist.

10. Bestrahlungsvorrichtung nach einem der Ansprüche 1 bis 9, dadurch gekennzeichnet, daß ein Zeitschaltwerk vorgesehen ist, das für die zeitliche Bestrahlungsdauer und/oder die Verstellung von Sendeleistungen programmierbar ist.

11. Bestrahlungsvorrichtung nach einem der Ansprüche 1 bis 10, dadurch gekennzeichnet, daß der Signalanalysator (11) patienteneigene, unregelmäßige Schwingungen (23) mit hohem Oberwellenanteil als pathologische Schwingungen erfaßt, daß solche pathologischen Schwingungen (23) an eine Invers-Schaltung (12) geleitet werden, in der sie invertiert werden und an den Endverstärker, ggfs. an die Modulationseinheit, weitergeleitet werden, so daß der Patient sofort mit der inversen pathologischen Schwingung für eine Auslöschung bestrahlt wird (Operationsblock 22).

12. Bestrahlungsvorrichtung nach einem der Ansprüche 1 bis 11, dadurch gekennzeichnet, daß der Signalanalysator (11) patienteneigene, regelmäßige Schwingungen als gesunde Schwingungen erfaßt, daß diese gesunden Schwingungen der Steuereinheit (13) zugeleitet werden und die Steuereinheit (13) die an der Niederfrequenzoszillatorstufe (2) eingestellte Niederfrequenz entsprechend der Frequenz und ggfs. der Amplitude der gesunden Schwingung nachregelt.

## Claims

1. An irradiation device for the treatment of living tissue with electromagnetic waves, with a high frequency oscillator stage for generating a high frequency wave train, in which arrangement the high frequency oscillator stage generates a frequency adjustable within the range of 100 to 200 MHz, with a low frequency oscillator stage which generates an adjustable frequency, with a modulation unit to which the output of the high frequency oscillator stage and the output of the low oscillator frequency stage are passed, so that a modulated wave train is obtained, and with an output amplifier to which a transmission antenna may be connected, in whose irradiation zone there may be positioned the tissue, or a patient, to be treated, characterized in that the frequency adjustment of the low frequency oscillator stage (2) is performed in a manner controllable by a connected control unit (13) within a frequency range of 1 Hz to 100 KHz, preferably up to 10 KHz, that by means of at least one sensor (10) known per se, oscillations (23) specific to the patient can be picked up, that the sensor (10) is followed down the line by a signal analyser (11) for assessing the oscillations (23) specific to the patient, which registers, in particular, alterations of these oscillations, that after the irradiation device has been started, the control unit (13) operates a (partial) run through the frequencies (processing block 16) starting from the top or bottom through the low frequency range, if required by means of a timer (14), in a controlled manner,

that the signal analyser (11) is connected to the control unit (13) and that, if an alteration of the oscillations is ascertained which corresponds to a response of the patient (8) to a certain frequency just radiated in the run through the frequencies, it passes a stop signal to the control unit (13), and

that, after receiving the stop signal, the control unit (13) stops the run through the frequencies, so that the frequency just being applied is maintained for the further irradiation process.

2. An irradiation device according to claim 1, characterized in that EEG sensors, ECG sensors or skin resistance sensors are used as the sensors (10) for detecting the oscillations specific to the patient, which register the oscillations according to their frequency and amplitude.

3. An irradiation device according to claim 1 or 2, characterized in that for checking the irradiation frequency retained, a run through the frequencies is again operated or, if required several runs are operated successively, by the timer (14) during an irradiation period (processing block 20), so that possible changes in the optimum response frequency of the patient (8) during the irradiation process are taken into account and are automatically allowed for.

4. An irradiation device according to one of claims 1 to 3, characterized in that a clock oscillator (5) is disposed as a chopper between the modulation unit (3) and the output amplifier (6), so that the modulated wave train is passed to the clock oscillator (5), and that the clock oscillator (5) can be set to a clock frequency ranging from 0.5 Hz to 40 Hz.

5. An irradiation device according to claim 4, characterized in that the ratio between the period of the wave train passed at the clock oscillator (5) to the interval time is 1 : 1 or less.

6. An irradiation device according to one of claims 1 to 5, characterized in that the length of the transmission antenna (7) corresponds to the set high frequency or its wave length λ, or whole fractions such as λ/2, λ/4 etc.

7. An irradiation device according to one of claims 1 to 6, characterized in that the high frequency oscillator stage (1) generates a frequency of 140 to 160 MHz.

8. An irradiation device according to one of claims 1 to 7, characterized in that it comprises a second low frequency oscillator stage which generates a frequency that can be set in the range of 1 Hz to 100 KHz, preferably to 10 KHz,

that the second low frequency oscillator stage is followed down the line by a second output amplifier to which a coil may be connected as an irradiation element, in which arrangement the tissue, or a patient, to be treated can be simultaneously brought into the irradiation range of the transmission antenna (7) and into the irradiation range of the coil.

9. An irradiation device according to one of claims 1 to 8, characterized in that the transmission power at the output amplifier (6) can be set from 0.5 mW to 100 mW, preferably 10 mW.

10. An irradiation device according to one of claims 1 to 9, characterized in that provision is made for a time switch which can be programmed for the temporal irradiation period and/or the adjustment of the transmission powers.

11. An irradiation device according to one of claims 1 to 10, characterized in that the signal analyser (11) registers irregular oscillations (23) specific to the patient with a high harmonic content as pathological oscillations,

that such pathological oscillations (23) are passed into an impedance inverter circuit (12) in which they are inverted and are passed onto the output amplifier and if required, to the modulation unit, so that the patient is immediately irradiated with the inverse pathological oscillation for their extinction (processing block 22).

12. An irradiation device according to one of claims 1 to 11, characterized in that the signal analyser (11) registers regular oscillations specific to the patient as healthy oscillations,

that these healthy oscillations are passed to the control unit (13) and that the control unit (13) readjusts the low frequency set at the low frequency stage (2) according to the frequency, and if required, the amplitude of the healthy oscillation.

**Revendications**

1. Dispositif d'irradiation pour le traitement de tissu vivant par des ondes électromagnétiques, comprenant

un étage oscillateur haute fréquence pour la production d'un train d'ondes haute fréquence, ledit étage oscillateur haute fréquence produisant une fréquence réglable dans une gamme de 100 à 200 MHz,

un étage oscillateur basse fréquence, qui produit une fréquence réglable,

un bloc de modulation, auquel sont reliées la sortie de l'étage oscillateur haute fréquence et la sortie de l'étage oscillateur basse fréquence, de telle sorte qu'un train d'ondes modulées est engendré, et

un amplificateur final auquel peut être raccordée une antenne émettrice, dans la zone de rayonnement de laquelle le tissu à traiter ou un patient peut être placé,

caractérisé en ce que,

le réglage de la fréquence de l'étage oscillateur basse fréquence (2) est réalisé de telle façon que la fréquence soit réglable dans une gamme de 1 Hz à 100 KHz, de préférence à 10 KHz, par un bloc de commande (13) qui y est connecté,

les oscillations (23) propres au patient peuvent être captées par au moins un capteur (10) connu en soi,

un analyseur de signal (11) destiné à évaluer les oscillations (23) propres au patient est connecté en série avec le capteur (10) et détecte en particulier des variations de ces oscillations,

après une mise en marche du dispositif d'irradiation, le bloc de commande (13) effectue un balayage de fréquence (en partie) (bloc fonctionnel 16), en commençant à partir du bas ou du haut, dans la gamme des basses fréquences, éventuellement sous la commande d'une horloge (14),

l'analyseur de signal (11) est relié au bloc de commande (13) et délivre un signal d'arrêt au bloc de commande (13) lorsqu'il se produit une variation déterminée des oscillations, ce qui correspond à une réponse du patient (8) à une fréquence rayonnée déterminée et précise pendant le balayage de fréquence, et

après réception du signal d'arrêt, le bloc de commande (13) arrête le balayage de fréquence, de sorte que ladite fréquence précise est conservée pour le processus ultérieur d'irradiation.

2. Dispositif d'irradiation selon la revendication 1, caractérisé en ce que, à titre de capteurs (10) pour détecter les oscillations propres au patient, on utilise des capteurs pour EEG (électro-encéphalogramme), des capteurs pour ECG (électrocardiogramme) ou des capteurs de mesure de la résistance de la peau, qui détectent les oscillations selon la fréquence et l'amplitude.

3. Dispositif d'irradiation selon la revendication 1 ou 2, caractérisé en ce que, pendant un temps d'irradiation, afin de vérifier la fréquence d'irradiation retenue, un ou éventuellement plusieurs balayages de fréquence sont à nouveau exécutés l'un après l'autre (bloc fonctionnel 20) par l'horloge (14), de sorte que des variations possibles de la fréquence de réponse optimale du patient (8) pendant le processus d'irradiation soient prises en considération et rajustées automatiquement.

4. Dispositif d'irradiation selon l'une quelconque des revendications 1 à 3, caractérisé en ce qu'un générateur d'impulsions d'horloge (5) est connecté en tant que hacheur entre le bloc de modulation (3) et l'amplificateur final (6), de sorte que le train d'ondes modulées soit appliqué au générateur d'impulsions d'horloge (5), et le générateur d'impulsions d'horloge (5) est réglable en fréquence d'horloge de 0,5 Hz à 40 Hz.

5. Dispositif d'irradiation selon la revendication 4, caractérisé en ce que le rapport entre la durée du train d'ondes transmis par le générateur d'impulsions d'horloge (5) et le temps de pose est de 1:1 ou plus petit.

6. Dispositif d'irradiation selon l'une quelconque des revendications 1 à 5, caractérisé en ce que la longueur de l'antenne émettrice (7) correspond à la haute fréquence réglée ou à sa longueur d'onde λ ou une fraction exacte telle que λ/2, λ/4, etc.

7. Dispositif d'irradiation selon l'une quelconque des revendications 1 à 6, caractérisé en ce que l'étage oscillateur haute fréquence (1) produit une fréquence comprise entre 140 et 160 MHz.

8. Dispositif d'irradiation selon l'une quelconque des revendications 1 à 7, caractérisé en ce qu'il comporte un second étage oscillateur basse fréquence, qui produit une fréquence réglable dans une gamme de 1 Hz à 100 KH, de préférence a 10 KHz, et en ce qu'un second amplificateur final, auquel une bobine peut être raccordée en tant qu'élément rayonnant, est connecté en serie avec le second étage oscillateur basse fréquence, le tissu à traiter ou un patient pouvant être amené simultanément dans la zone de rayonnement de l'antenne émettrice (7) et dans la zone de rayonnement de la bobine.

9. Dispositif d'irradiation selon l'une quelconque des revendications 1 à 8, caractérisé en ce que la puissance d'émission est réglable au niveau de l'amplificateur final (6) de 0,5 mW à 100 mW, de préférence 10 mW.

10. Dispositif d'irradiation selon l'une quelconque des revendications 1 à 9, caractérisé en ce qu'il est prévu une minuterie qui est programmable pour régler la durée d'irradiation et/ou la puissance d'émission.

11. Dispositif d'irradiation selon l'une quelconque des revendications 1 à 10, caractérisé en ce que l'analyseur de signal (11) analyse des oscillations irrégulières (23), propres au patient, qui ont de hautes harmoniques, comme étant des oscillations pathologiques,
et en ce que de telles oscillations pathologiques sont appliquées à un intégrateur inverse (12) dans lequel elles sont inversées, et elles sont transmises à l'amplificateur final, éventuellement au bloc de modulation, de telle sorte que le patient soit immédiatement irradié avec les oscillations pathologiques inverses en vue d'une neutralisation (bloc fonctionnel 22).

12. Dispositif d'irradiation selon l'une quelconque des revendications 1 à 11, caractérisé en ce que l'analyseur de signal (11) analyse des oscillations régulières, propres au patient, comme étant des oscillations saines,
et en ce que ces oscillations saines sont appliquées au bloc de commande (13), et le bloc de commande (13) rajuste la basse fréquence réglée au niveau de l'étage oscillateur basse fréquence (2), en correspondance avec la fréquence et éventuellement l'amplitude des oscillations saines.

FIG.1

EP 0 500 983 B1

Start _14_

HF-Einstellung _15_

Start: Zeitgeber für
NF-Durchlauf _16_

_17_

Reaktion
Patient      *nein*

*ja*

_18_

_21_

erreichte NF-
Frequenz wird
beibehalten _19_

Bestrahlung mit
inversen, pathologischen Schwingungen

_22_

nach bestimmter Zeit
erfolgt Überprüfung _20_

FIG. 2

FIG. 3